(19) Europäisches Patentamt / European Patent Office / Office européen des brevets

(11) **EP 3 010 516 B1**

(12) **FASCICULE DE BREVET EUROPEEN**

(45) Date de publication et mention de la délivrance du brevet:
**26.09.2018 Bulletin 2018/39**

(21) Numéro de dépôt: **14731686.3**

(22) Date de dépôt: **14.05.2014**

(51) Int Cl.:
*A61K 35/64* (2015.01)        *A61K 31/728* (2006.01)
*A61P 17/02* (2006.01)

(86) Numéro de dépôt international:
**PCT/FR2014/051123**

(87) Numéro de publication internationale:
**WO 2014/202851 (24.12.2014 Gazette 2014/52)**

(54) **COMPOSITION CICATRISANTE ET UTILISATION**

HEILUNGSZUSAMMENSETZUNG UND VERWENDUNG DAVON

HEALING COMPOSITION AND USE THEREOF

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **20.06.2013 FR 1355810**

(43) Date de publication de la demande:
**27.04.2016 Bulletin 2016/17**

(73) Titulaire: **Melipharm**
**87000 Limoges (FR)**

(72) Inventeurs:
- **QUERO, Fabien**
  **F-33110 Le Bouscat (FR)**
- **MALEPEYRE, Carmen**
  **F-87430 Verneuil sur Vienne (FR)**
- **RAYNAUD, Anaïs**
  **F-87410 Le Palais sur Vienne (FR)**
- **PETIT, Jean-Michel**
  **F-87170 Isle (FR)**

(74) Mandataire: **Aquinov**
**Allée de la Forestière**
**33750 Beychac et Caillau (FR)**

(56) Documents cités:
CH-A1- 699 182        US-A1- 2003 021 834
US-A1- 2011 104 279    US-B1- 6 630 442

- **COOPER R A ET AL: "Antibacterial activity of honey against strains of Staphylococcus aureus from infected wounds", JOURNAL OF THE ROYAL SOCIETY OF MEDICINE, vol. 92, no. 6, 1 juin 1999 (1999-06-01), pages 283-285, XP008092256, ROYAL SOCIETY OF MEDICINE, LONDON, GB ISSN: 0141-0768**
- **ALANDEJANI T ET AL: "Effectiveness of honey on Staphylococcus aureus and Pseudomonas aeruginosa biofilms", OTOLARYNGOLOGY AND HEAD AND NECK SURGERY, vol. 141, no. 1, 1 juillet 2009 (2009-07-01), pages 114-118, XP026251754, ROCHESTER, US ISSN: 0194-5998, DOI: 10.1016/J.OTOHNS.2009.01.005 [extrait le 2009-03-09]**
- **MOLAN P C: "Potential of honey in the treatment of wounds and burns", AMERICAN JOURNAL OF CLINICAL DERMATOLOGY, vol. 2, no. 1, 1 février 2001 (2001-02-01) , pages 13-19, XP009168009, ADIS, US ISSN: 1175-0561**
- **SOLDATI D ET AL: "Mucosal wound healing after nasal surgery. A controlled clinical trial on the efficacy of hyaluronic acid containing cream", DRUGS UNDER EXPERIMENTAL AND CLINICAL RESEARCH, vol. 25, no. 6, 1999, pages 253-261, XP009172538, ISSN: 0378-6501**
- **LEON-VILLAPALOS J ET AL: "Topical management of facial burns", BURNS, vol. 34, no. 7, 1 novembre 2008 (2008-11-01), pages 903-911, XP025479836, BUTTERWORTH HEINEMANN, GB ISSN: 0305-4179, DOI: 10.1016/J.BURNS.2008.01.025 [extrait le 2008-06-05]**

- **DAVIS S C ET AL: "Cosmeceuticals and natural products: wound healing", CLINICS IN DERMATOLOGY, vol. 27, no. 5, 1 septembre 2009 (2009-09-01), pages 502-506, XP026471181, J.B. LIPPINCOTT, PHILADELPHIA, PA, US ISSN: 0738-081X, DOI: 10.1016/J.CLINDERMATOL.2009.05.015 [extrait le 2009-08-18]**
- **SINGH ONKAR ET AL: "Collagen dressing versus conventional dressings in burn and chronic wounds: a retrospective study.", JOURNAL OF CUTANEOUS AND AESTHETIC SURGERY , janvier 2011 (2011-01), page 3PP, XP002729702, Extrait de l'Internet: URL:http://www.jcasonline.com/article.asp? issn=0974-2077;year=2011;volume=4;issue=1; spage=12;epage=16;aulast=Singh [extrait le 2014-09-15]**

**Description**

La présente invention concerne une composition particulière constituée de miel et au moins un constituant de la matrice extracellulaire et/ou au moins un béta-glucane, et son utilisation sur la peau ou les muqueuses pour une activité cicatrisante et/ou antibactérienne. Une plaie est une rupture de la barrière cutanée, qui, outre la lésion d'organes sous-jacents, peut entraîner la pénétration d'agents infectieux dans l'organisme. Les tissus humains et animaux sont capables de réparer une plaie par des processus de réparation qui leurs sont propres, et la cicatrisation est un phénomène biologique naturel. Cette capacité reste cependant soumise à de nombreuses variations, la rapidité et la qualité de la cicatrisation d'une plaie dépendant de l'état général de l'organisme atteint, de l'état et de la localisation de la plaie, et de la survenue ou de l'absence d'une infection.

Aussi, en cas de lésion cutanée, il est nécessaire de recourir à l'application de produits favorisant la cicatrisation. Il existe actuellement plusieurs produits cicatrisants.

Certains de ces produits cicatrisants agissent en maintenant un environnement humide pour favoriser la cicatrisation, comme par exemple des hydrocolloïdes constitués de polymères absorbants et d'autres gélifiants, des alginates, constitués d'alginate de calcium et de sodium, des hydrogels, des pansements absorbants ou des pansements à l'acide hyaluronique. Ces produits, même s'ils favorisent la cicatrisation en générant un environnement humide, ne présentent pas de propriétés antibactériennes. Des variantes de ces produits à base d'argent ou d'iode ont donc été développées pour contrecarrer cette lacune. Cependant, bien que très antibactériens les pansements à l'argent présentent souvent l'inconvénient d'être cytotoxiques. Ils induisent la mort cellulaire des cellules impliquées dans la cicatrisation des plaies. Il est par conséquent, déconseillé d'utiliser les pansements à base d'argent sur de longues périodes en raison de leur cytotoxicité. Quant aux produits à base d'iode ils sont également cytotoxiques et leur activité antimicrobienne diminue fortement en présence de matière organique (pus, fibrine ou nécrose). De plus, il existe des risques de sensibilisation avec eczéma de contact et allergie à ces produits.

C'est pourquoi, la présente invention a pour objectif de proposer une composition à base de miel palliant les inconvénients de l'art antérieur, simple de fabrication, économique, facile d'application et générant à la fois un environnement humide et une activité antibactérienne afin de réunir les conditions favorables à une cicatrisation rapide et efficace sans générer la toxicité des produits précités.

A cet effet, l'invention vise une composition comprenant un mélange de miel et d'au moins un constituant de la matrice extracellulaire choisi parmi le collagène, l'élastine et les glycosaminoglycanes en particulier l'acide hyaluronique et/ou d'au moins un béta-glucane, pour son utilisation comme produit de santé cicatrisant et/ou antibactérien à application topique.

L'utilisation de pansements et de soins au miel pour le traitement de certaines infections au niveau des plaies est connue. On sait en particulier que le miel présente des propriétés antibactériennes et antifongiques et qu'il permet de réduire le temps de cicatrisation, sans infection ni effets secondaires.

Plusieurs documents décrivent l'utilisation du miel pour le traitement des plaies, comme notamment les brevets CH699182, us6630442, US2003/021834 ou US2011/10427 mais ces produits ne sont pas efficaces comme produit cicatrisant ou antibactérien. C'est le cas également des publications : Cooper R A et al. «Antibacterial activity of honey against strains of Staphylococcus aureus from infected wounds » Journal of the Royal Society of Medecine,London GB, vol. 92, no. 6, 1 juin 1999 ; Alandejani T et al. « Effectiveness of honey on Staplylococcus aureus and Pseudomonas aeruginosa biofilms » Otolaryngology and head and neck surgery, Rochester, US, vol. 141, no. 1, 1juillet 2009 ; Molan P C « Potential of honey in the treatment of wounds and burns » American Journal of Clinical Dermatology, ADIS, US, vol. 2, no. 1, 1 fevrier 2001.

De plus, d'autres solutions de cicatrisations ont été décrites dans les publications suivantes : Solidati D et al « Mucosal wound healing after nasal surgery. A controlled clinical trial on the efficacy of hyaluronic acid containing cream », Drugs under experimental and clinical research, vol. 25, no. 6, pages 253-261 ; Leon-Villapalos J et al : « Topical management of facial burns », Burns, Butterworth Heinemann, GB, vol. 34, no. 7, 1 novembre 2008, pages 903-911 ; Davis S C et al. : « Cosmeceuticals and natural products : wound healing », Clinics in Dermatology, J.B. Lippincott, Philadelphia, PA, US, vol. 27, no. 5, 1 septembre 2009 ; Singh Onkaret al : « Collagen dressing versus conventional dressings in burn and chronic wounds : a rétrospective study », Journal of Cutaneousand Aesthetic Surgery Jan 2011, vol. 4, no. 1, janvier 2011 ; mais les solutions proposées ne sont pas satisfaisantes.

Avantageusement, en comparaison aux produits décrits dans ces documents, le mélange selon l'invention présente un effet synergique important et surprenant, qui permet de proposer une composition cicatrisante, réparatrice et antibactérienne, avec une bonne tolérance et sans effets secondaires notoires, et avec des propriétés qui dépassent celles des produits existants actuellement.

D'autres caractéristiques et avantages ressortiront de la description en détails de l'invention qui va suivre.

L'invention vise donc une composition pour son utilisation comme produit de santé cicatrisant et/ou antibactérien à application topique sur la peau ou les muqueuses, comprenant :

- entre 5% et 99,8 % de miel, et
- au moins un constituant de la matrice extracellulaire choisi parmi le collagène, l'élastine et les glycosaminoglycanes dont l'acide hyaluronique qui s'il est compris dans la composition est présent à raison de 0,05 à 10 %, et/ou au moins un béta-glucane,

Les pourcentages étant donnés en poids par rapport au poids de matière sèche de la composition

Par miel au sens de l'invention, on entend un miel naturel ou un miel artificiel, ou encore un mélange de plusieurs miels naturel(s) et/ou artificiel(s).

Par miel artificiel on entend au moins un sucre. Il peut s'agir également de la combinaison d'au moins deux sucres.

Préférentiellement, le miel est un miel naturel choisi parmi les miels de thym, de miellat, de sarrasin, de manuka et leurs mélanges. Il peut s'agir par exemple d'un miel tel que décrit dans la demande FR1258722.

Si le miel utilisé dans la composition est un miel artificiel, il s'agit préférentiellement d'un mélange saccharidique comprenant majoritairement du glucose et/ou du fructose et/ou du saccharose.

La composition selon l'invention, en plus du miel comprend :

- au moins un constituant de la matrice extracellulaire choisi parmi le collagène, l'élastine et les glycosaminoglycanes tels que l'acide hyaluronique, les héparanes sulfates, les kératanes sulfates ou les chondroïtines sulfates, et/ou
- au moins un béta-glucane.

Les béta-glucanes présents dans la composition sont des polysaccharides constitués d'unités glucose reliées par des liaisons $\beta(1,3)$ et/ou $\beta(1,4)$ et/ou $\beta(1,6)$.

Il peut s'agir par exemple de béta-glucanes ayant un poids moléculaire compris entre $0,5.10^6$ Da et $1.10^6$ Da.

Les glycosaminoglycanes présents dans la composition peuvent être choisis parmi l'acide hyaluronique, les héparanes sulfates, les kératanes sulfates et/ou les chondroïtines sulfates. L'acide hyaluronique présent dans la composition est préférentiellement un sel d'acide hyaluronique ou un dérivé d'acide hyaluronique encore plus préférentiellement un sel sodique d'acide hyaluronique de poids moléculaire supérieur à 20 kDa.

Selon un mode de réalisation adapté l'acide hyaluronique présent dans la composition présente un poids moléculaire supérieur à 100 000 Da.

Le collagène présent dans la composition est préférentiellement un peptide de collagène issu d'hydrolysat de collagènes de poisson, de porc ou de boeuf ou un peptide biomimétique de collagène.

L'élastine présente dans la composition est préférentiellement un hydrolysat de tendon de boeuf ou un biomimétique d'élastine.

Selon un mode de réalisation particulièrement adapté, la composition comprend entre 5% et 99,8% de miel en poids de matière sèche de la composition.

Si la composition comprend de l'acide hyaluronique, il est présent entre 0,05% et 10% en poids de matière sèche de la composition, encore plus préférentiellement entre 0,05% et 1%.

Si la composition comprend du collagène, il est présent entre 0,001% et 50% en poids de matière sèche de la composition, encore plus préférentiellement entre 0,001% et 5%.

Si la composition comprend de l'élastine, il est présent entre 1% et 25% en poids de matière sèche de la composition, encore plus préférentiellement entre 5% et 10%.

Si la composition comprend un glycosaminoglycane autre que l'acide hyaluronique, il est présent entre 0,1% et 20% en poids de matière sèche de la composition, encore plus préférentiellement entre 0,1% et 10%.

Si la composition comprend un béta-glucane, il est présent entre 0,01% et 10% en poids de matière sèche de la composition, encore plus préférentiellement entre 0,01% et 5%.

La composition peut être composée exclusivement :

- de miel, et
- de collagène et/ou d'élastine et/ou de glycosminoglycanes (notamment d'acide hyaluronique) et/ou de béta-glucane, ou d'un mélange de ceux-ci.

Elle peut également contenir en plus de la lactoferrine de la glucose-oxydase, de l'oxyde de zinc, de la lactoperoxydase, des thiocyanates, du lysozyme, du xylitol, de la vanilline, des sucres tels que galactose, dextrose, rhamnose, mannose, téflose®, des oligosaccharides, de l'acide lactobionique, du bisabolol, de l'allantoïne, de l'aloe vera, de la propolis, du squalane, des vitamines, du panthénol, des céramides, du bentonite et/ou du kaolin.

Elle peut aussi contenir des excipients choisis parmi les excipients dermatologiquement compatibles et/ou applicables sur les muqueuses afin d'obtenir une composition liquide telle qu'une lotion, ou semi-solide tels qu'une crème, une pommade, un spray, un gel, une pâte, un suppositoire, un ovule ou une poudre. Il peut s'agir par exemple d'excipients tels que des sucres et dérivés de sucres, des polysaccharides (pectines, amidon et dérivés, alginates et dérivés, chitosan et dérivés, dérivés de cellulose, des gommes), des polymères de synthèse, des cires, des huiles naturelles ou non, des beurres, des cires, des produits minéraux (silice, talc, argiles, oxyde de titane), des glycérides et autres esters gras, des tensio-actifs, l'eau, l'éthanol, le propylène glycol, le butylène glycol, le polyéthylène glycol, le glycérol, le sorbitol, des hydrocarbures et silicones, des protéines et peptides et autres excipients connus de l'homme de métier.

La composition peut aussi contenir des additifs auxiliaires de formulation tels que des tensioactifs, des agents gélifiants, des agent absorbants, des agents humectants, des solvants, des agents d'étalement, des stabilisants, des complexants, des modificateurs rhéologiques, des conservateurs, des anti-oxydants et antimicrobiens, des colorants et des parfums.

Selon une variante particulièrement adaptée de l'invention, la composition comprend en plus du miel, au moins de l'acide hyaluronique et au moins un béta-glucane.

La composition selon l'invention peut être stérile, c'est-à-dire qu'elle a subi un procédé de stérilisation. La stérilisation est préférentiellement réalisée par rayonnement gamma.

La composition selon l'invention peut être obtenue par simple mélange des constituants. Préférentiellement, si la composition contient :

- un glycosaminoglycane : elle est obtenue par la dispersion d'une poudre (par exemple une poudre fine d'acide hyaluronique) ou d'une solution (par exemple une solution d'acide hyaluronique) dans une composition de miel,
- un béta-glucane : elle est obtenue par dispersion du béta-glucane dans une composition de miel soit sous forme de poudre soit sous forme d'une solution de béta-glucane,
- du collagène : elle est obtenue par ajout du collagène dans une composition de miel soit sous forme de poudre soit sous forme d'une solution,
- de l'élastine : elle est obtenue par dispersion d'une solution d'élastine dans une composition de miel.

Si le procédé est un procédé à chaud, la température de chauffe du miel ou du mélange contenant du miel doit être inférieure à 40°C.

La composition selon l'invention peut se présenter en poudre ou sous différentes formes liquides ou semi-liquides, notamment lotion, crème, émulsion, gel, pommade, spray, ovule vaginal, suppositoires.

La composition est utilisée comme produit cicatrisant et/ou antibactérien. En effet, le mélange de miel et de béta-glucane et/ou de composant de la matrice extracellulaire, agit en synergie pour à la fois :

- augmenter la prolifération et la migration des cellules de la peau, en particulier des fibroblastes et des kératinocytes,
- diminuer la charge bactérienne, et ainsi favoriser indirectement la cicatrisation.

Les effets obtenus sont bien meilleurs que ceux obtenus avec le miel seul ou les béta-glucanes seuls ou un composant de la matrice extracellulaire seul. Il existe un effet synergique surprenant. Au niveau cellulaire, le miel seul ou les béta-glucanes seuls ou les composants de la matrice extracellulaire seuls permettent d'augmenter la prolifération des fibroblastes et des kératinocytes, mais lorsqu'ils sont utilisés ensemble, l'augmentation de la prolifération des fibroblastes et des kératinocytes est plus importante que la somme des proliférations induites par les constituants seuls (voir notamment les résultats d'essais des points 3 à 6). De plus, au niveau bactérien, ni l'acide hyaluronique, ni les béta-glucanes, ni le miel lorsqu'ils sont fortement dilués, ne présentent une activité antibactérienne. Cependant, l'association des constituants selon l'invention, en particulier l'association de l'acide hyaluronique avec le miel ou de béta-glucane avec le miel ou de l'acide hyaluronique et de béta-glucane avec le miel, à la même concentration montre une inhibition importante de la prolifération bactérienne (voir tableau 1).

Selon l'invention, la composition peut donc être avantageusement utilisée comme produit de santé à application topique sur la peau ou les muqueuses chez l'homme ou l'animal, très préférentiellement comme:

- dispositif médical destiné à être appliqué sur des irritations ou des lésions cutanées notamment pour :

  • le traitement des brûlures de premier et second degré,
  • le traitement et/ou la prévention des désunions post opératoires de cicatrice,
  • le traitement et/ou la prévention post-opératoire des cavités résiduelles des sinus pilonidaux,
  • le traitement des cicatrices chirurgicales infectées après mise à plat,
  • le traitement des ulcères et escarres,
  • le traitement des plaies traumatiques et/ou chirurgicales,
  • le traitement des plaies aigües et chroniques,
  • le traitement des plaies cancérologiques,

- le traitement des emplacements de stomies,
- le traitement des dermabrasions,
- le traitement des plaies superficielles.

- une composition, par exemple un dispositif médical ou une composition dermatologique, pour le traitement des dermatites, de l'acné ou des érythèmes fessiers, impétigo, folliculites, furoncle, panaris, mycoses,
- gel buccal, notamment destiné à traiter les aphtes et les mucites,
- stick ou baume à lèvre destiné à traiter les gerçures,
- crème pour les mains et les pieds destinée notamment au traitement des crevasses et des engelures,
- baume destiné à la prévention, au soin et/ou au traitement des crevasses du mamelon,
- gel vaginal,
- gel rectal, suppositoire ou crème rectale.

Par dispositif médical on entend un produit destiné à être utilisé chez l'homme ou l'animal à des fins notamment de prévention, de contrôle, de traitement et/ou d'atténuation d'une maladie ou d'une blessure.

S'il s'agit d'un dispositif médical pour la cicatrisation de lésions cutanées se présentant sous forme de poudre, spray, gel, crème ou pommade, la composition selon l'invention peut en particulier s'appliquer selon le protocole d'utilisation suivant :

- rincer la plaie et sécher doucement,
- recouvrir l'intégralité de la plaie ou le pansement secondaire d'une pellicule de la composition selon l'invention,
- recouvrir de compresses et d'un pansement occlusif durant les premières applications pour les plaies très exsudatives.

En phase de détersion, et en fonction de l'état de la plaie, il est conseillé de refaire le pansement une à deux fois par 24 heures.

En phase de bourgeonnement, il est conseillé de refaire le pansement toutes les 48 à 72 heures.

En phase d'épithélialisation, il est conseillé de refaire le pansement tous les 3 ou 4 jours. L'invention est à présent illustrée par des exemples de composition et des résultats d'essais démontrant son efficacité.

## A. Exemples de composition selon l'invention

### 1. Exemple 1

La composition de l'exemple 1 est un gel hyperosmotique. Elle est constituée de :

- 99,8% d'un mélange de miels de thym, miellat, sarrasin et manuka,
- 0,2% de hyaluronate de sodium.

les pourcentages étant donnés en poids par rapport au poids total de matière sèche de la composition.

La composition est obtenue par mélange des deux composés.

### 2. Exemple 2

La composition de l'exemple 2 est un pommade constituée de :

- qsp 100% de miel de thym,
- 25% de triglycérides,
- 11% de cire d'abeille,
- 7,5% de beurre de karité,
- 1,83% de tocophérol acétate,
- 5% de glyceryl stearate,
- 0,2% de hyaluronate de sodium,
- 0,07% de tocophérol.

les pourcentages étant des pourcentages en poids par rapport au poids total de la matière sèche de la composition.

La composition est obtenue par la mise en oeuvre des étapes suivantes :

- disperser finement le hyaluronate de sodium dans le miel,
- chauffer le mélange à moins de 40°C,
- chauffer la phase lipophile (cire, beurre et glycéride) à environ 70°C pour faire fondre les solides,
- quand la phase lipophile a atteint la température de 40°C, ajouter le tocophérol acétate et le tocophérol et le mélange de miel et de hyaluronate de sodium,
- mélanger la pommade jusqu'à refroidissement.

### 3. Exemple 3

La composition de l'exemple 3 est une crème constituée de :

- qsp 100% de miel artificiel,
- 30% de glycérol,
- 10,3% de beurre de karité,
- 5% de cetearyl alcohol et cetearyl glucoside,
- 2% de cire d'abeille blanche,
- 1,82% de tocophérol acétate,
- 0,8% d'acide stéarique,
- 0,2% de hyaluronate de sodium,
- 0,07% de tocophérol,
- 0,01% de palmitate d'ascorbyle,

les pourcentages étant des pourcentages en poids par rapport au poids total de la matière sèche de la composition.
La composition est obtenue par la mise en oeuvre des étapes suivantes :

- disperser finement le hyaluronate de sodium dans le miel,
- chauffer le mélange à moins de 40°C,
- chauffer la phase lipophile (cire, beurre) et le glycérol séparément à environ 70°C,
- ajouter la phase lipophile au glycérol et quand ce mélange atteint environ 40°C, ajouter le tocophérol acétate, le tocophérol, le palmitate d'ascorbyle, puis le mélange de miel et de hyaluronate de sodium,
- mélanger la crème jusqu'à refroidissement.

### 4. Exemple 4

La composition de l'exemple 4 est un gel hyperosmotique. Elle est constituée de :

- 90% de miel de sarrasin,
- 9,8% de glycérol,
- 0,2% de hyaluronate de sodium.

les pourcentages étant des pourcentages en poids par rapport au poids total de la matière sèche de la composition.
La composition est obtenue par la mise en oeuvre des étapes suivantes :

- une solution de hyaluronate de sodium à 2% dans un mélange eau/glycérol/éthanol (dans un ratio 10/10/3) est préparée puis déshydratée par distillation sous vide ;
- cette solution de hyaluronate de sodium glycérolée est ensuite mélangée dans le miel à température ambiante.

### 5. Exemple 5

La composition de l'exemple 5 est un gel hyperosmotique. Elle est constituée de :

- 94,8% d'un mélange de miels de thym, miellat, sarrasin et manuka,
- 5% d'un collagène de poisson,
- 0,2% de hyaluronate de sodium.

les pourcentages étant des pourcentages en poids par rapport au poids total de la matière sèche de la composition.
La composition est obtenue par le mélange des trois composés.

## 6. Exemple 6

La composition de l'exemple 6 est un gel hyperosmotique. Elle est constituée de :

- 95% d'un mélange de miels de thym, miellat, sarrasin et manuka,
- 5% d'une solution de béta-glucanes.

les pourcentages étant des pourcentages en poids par rapport au poids total de la matière sèche de la composition.
La composition est obtenue par le mélange des deux composés.

## 7. Exemple 7

La composition de l'exemple 7 est un gel hyperosmotique. Elle est constituée de :

- 94,8% d'un mélange de miels de thym, miellat, sarrasin et manuka,
- 5% d'une solution de béta-glucanes,
- 0,2% de hyaluronate de sodium.

les pourcentages étant des pourcentages en poids par rapport au poids total de la matière sèche de la composition.
La composition est obtenue par le mélange des trois composés.

## B. Evaluation de l'efficacité de la composition selon l'invention

Des essais ont été réalisés pour démontrer l'efficacité cicatrisante et antibactérienne de plusieurs compositions selon l'invention d'une part et comparer cette efficacité à celle du constituants seuls.

## 1. Evaluation de l'activité antibactérienne de la composition selon l'invention et comparaison par rapport aux ingrédients seuls

L'objectif de cette étude est d'évaluer l'activité antibactérienne de la composition selon l'invention (exemples 1, 6 et 7), en comparaison à celle du miel seul. Les échantillons ont été testés à 3, 5, 7 et 9% v/v.
Le protocole opératoire est le suivant.
La prolifération bactérienne en présence des produits à tester est déterminée par une méthode en microplaque 96 puits. Chaque puits est inoculé avec 50 $\mu$L de suspension bactérienne à tester réalisée dans un bouillon Muller-Hinton (MH) + 150 $\mu$L de solution de produit à tester dilué. La concentration bactérienne dans le puits est fixée à $10^6$ CFU/mL. Le témoin positif (150 $\mu$L de suspension bactérienne + 50 $\mu$L de milieu MH) correspond au 100% de prolifération bactérienne. Le témoin négatif (200 $\mu$L de milieu de culture) correspond au 0% de prolifération bactérienne. Chaque échantillon est testé en triplicata. Les densités optiques (DO) sont lues une première fois au temps 0 (T0) à 450 nm. Les plaques sont ensuite incubées 24h à 37°C sous agitation. Au terme de l'incubation la DO est remesurée à 450 nm (T24). Le pourcentage de prolifération est calculé ainsi :
Prolifération = $(DO_{T24} - DO_{T0}) / (DO_{T24\ Témoin\ +} - DO_{T0\ Témoin\ +})$ Les résultats obtenus en pourcentage de prolifération bactérienne sont présentés dans le tableau 1 suivant.

*Tableau 1 : Evaluation de l'activité antibactérienne*

| | Concentration testées (en % v/v) | Pourcentage de prolifération bactérienne (*Staphylococcus aureus*) |
|---|---|---|
| Miel seul | 3 | 32% |
| | 5 | 25% |
| | 7 | 19% |
| | 9 | 15% |
| Composition exemple 1 | 3 | 25% |
| | 5 | 15% |
| | 7 | 13% |
| | 9 | 1% |

(suite)

|  | Concentration testées (en % v/v) | Pourcentage de prolifération bactérienne (*Staphylococcus aureus*) |
|---|---|---|
| Composition exemple 6 | 3 | 28% |
|  | 5 | 23% |
|  | 7 | 11% |
|  | 9 | 2% |
| Composition exemple 7 | 3 | 27% |
|  | 5 | 20% |
|  | 7 | 13% |
|  | 9 | 6% |

On constate que les compositions selon l'invention présentent une amélioration de l'activité antibactérienne. En outre, cette activité est plus importante que celle du miel seul.

**2. Evaluation de l'activité cicatrisante - prolifération cellulaire de l'acide hyaluronique**

L'objectif de cette étude est d'évaluer l'activité cicatrisante d'une composition selon l'invention, en comparaison à celle du miel seul et à celle de l'acide hyaluronique seul, et de vérifier la stabilité de la composition après stérilisation par rayonnement gamma.

Les échantillons testés sont les suivants :

- miel brut : miel de thym,
- acide hyaluronique testé seul à 0,5%,
- composition constituée de miel brut de thym dans lequel on a ajouté 0,2% ou 0,5% d'acide hyaluronique,
- composition constituée de miel brut de thym dans lequel on a ajouté 0,2% d'acide hyaluronique, et traitée par irradiation gamma (30kGy).

Les produits sont dilués pour obtenir une concentration de 5,12% v/v de l'échantillon.

Le protocole opératoire est le suivant :

Les cultures primaires de fibroblastes humains PAF 08052 sont ensemencées dans des microplaques 48 puits à raison de 10 000 cellules par puits avec un volume de milieu de culture complet de 500 $\mu$L. Les cellules vont ensuite adhérer au fond des puits durant 24 heures. Le milieu sera ensuite remplacé par la composition selon l'invention diluée dans du milieu de culture privé de sérum. La concentration testée est 1,3% v/v à raison de 500 $\mu$L par puits et incubé pendant 48 heures à 37°C + 5% $CO_2$. Les cellules sont ensuite décollées avec de la trypsine puis comptées sur lame de Malassez avec un colorant d'exclusion (bleu Trypan). Les résultats sont indiqués en nombre de cellules par puits.

Les résultats obtenus, sont présentés dans le tableau 2 suivant.

*Tableau 2 : Evaluation de l'activité cicatrisante*

|  | Nombre de cellules par puits |
|---|---|
| Miel seul | 13 333,3 |
| Acide hyaluronique 0,5% | 15 833,3 |
| Miel + Acide hyaluronique 0,2% | 20 833,3 |
| Miel + Acide hyaluronique 0,5% | 20 000 |
| Miel + Acide hyaluronique 0,2% + irradiation gamma | 20 000 |

On constate que la composition selon l'invention favorise la prolifération des fibroblastes et donc la cicatrisation et que cet effet est nettement amélioré en comparaison à celui du miel seul ou à celui de l'acide hyaluronique seul.

En outre, cette activité est maintenue même après stérilisation par rayonnement gamma.

## 3. Evaluation de l'effet synergique d'un mélange miel + acide hyaluronique

Ces essais ont pour objectif de démontrer l'effet synergique du mélange selon l'invention.
Le pourcentage de prolifération fibroblastique est déterminé par rapport à un témoin non traité avec la composition.

### al Test de Berenbaum

Le test de Berenbaum permet de déterminer des effets additifs, synergiques ou antagonistes entre deux produits mis en mélanges aux contacts de cellules (Berenbaum, 1977). Ce test fut développé afin de déterminer à l'aide de données expérimentales insérées dans une formule mathématique l'impact de plusieurs composés mélangés. Il est applicable à de nombreux domaines de la biologie.

Ce test est appliqué ici au miel et à l'acide hyaluronique (AH). Pour cela, des gammes d'AH et de miel sont utilisées pour stimuler les fibroblastes durant 48 h. Le miel et l'AH sont mis dans le milieu de culture à des concentrations inférieures au maximum de stimulation. Après 48 h de stimulation, les cellules sont dénombrées et le pourcentage de prolifération est calculé. L'analyse des résultats de dénombrements se fait sur la base d'une recherche d'activité équivalente pour chaque composé. Par exemple, l'activité recherchée est prolifération de 150%. La dose de miel nécessaire pour obtenir 150% de prolifération (dans le cas de notre exemple) est désignée par Aeq pour le miel. Beq désigne la dose nécessaire pour obtenir le même effet (150% de prolifération) avec l'AH. Cette recherche de concentrations équivalentes se fait grâce à la réalisation de la gamme de miel seul et de la gamme d'AH seul.

Dans un second temps, le mélange des deux composés permet de déterminer les doses de chaque produit à introduire ensemble pour obtenir le même effet (150% de prolifération) qu'avec Aeq ou Beq. On désigne alors la dose nécessaire de miel comme dose de A et celle d'AH comme dose de B.

$$\frac{\text{Dose de A}}{\text{Aeq}} + \frac{\text{Dose de B}}{\text{Beq}}$$

Par la suite, il est possible d'introduire ces données dans l'équation suivante :
Si le résultat de cette équation est égal à 1, l'effet des 2 composés sera additif. Si le résultat est inférieur à 1, nous pourrons parler de synergie. En revanche si le résultat est supérieur à 1 les composés sont désignés comme antagonistes.

### b) Réalisation d'une gamme d'acide hyaluronique (AH)

Durée du traitement = 48 h

Cellules testées = fibroblastes

Les concentrations testées correspondent aux concentrations en acide hyaluronique retrouvées dans les puits de culture une fois les produits des exemples dilués. Ces dilutions sont nécessaires pour la réalisation de ce test.

*Tableau 3*

| Quantité d'acide hyaluronique (%) | 0% | 0,00023% | 0,00047% | 0,00094% | 0,0018% | 0,0037% | 0,0075% | 0,015% |
|---|---|---|---|---|---|---|---|---|
| Pourcentage de prolifération (%) | 100% | 100% | 109% | 127% | 182% | 182% | 182% | 190% |

*c) Réalisation d'une gamme de miel de thym*

Durée du traitement = 48 h

Cellules testées = fibroblastes

*Tableau 4*

| Quantité de miel (%) | 0% | 0,3% | 0,6% | 1,28% | 2,56% |
|---|---|---|---|---|---|
| Pourcentage de prolifération (%) | 100% | 138% | 145% | 154% | 163% |

*d) Réalisation de mélanges miel de thym + AH*

Durée du traitement = 48 h

Cellules testées = fibroblastes

*Tableau 5: Bilan des résultats obtenus et calcul des concentrations à utiliser dans la composition - Bilan des concentrations ayant des effets synergiques*

| Pourcentages de composition testés | Quantité d'AH calculée, dans la composition avec une quantité de miel de l'ordre de 100% | Miel (environ 100%) (% de prolifération) | Acide hyaluronique (% de prolifération) | Miel + Acide hyaluronique (% de prolifération) | Indice de Berenbaum |
|---|---|---|---|---|---|
| Testé à 0,6% | 0,05% | 145,5% | 100% | 173% | 0,36 |
| Testé à 0,30% | 0,1% | 138,2% | 100% | 167% | 0,24 |
| Testé à 0,3% | 0,2% | 138,2% | 109% | 164% | 0,28 |
| Testé à 0,6% | 0,2% | 145,5% | 127% | 180% | 0,75 |

Pour chacune des compositions comportant entre 0,05% et 0,2% d'acide hyaluronique, l'indice de Berenbaum est inférieur à 1. Ce résultat indique une synergie entre le miel et l'acide hyaluronique induisant une augmentation de la prolifération des fibroblastes à ces concentrations.

*e) Résultats sur la stimulation de la synthèse de collagène III*

Le collagène III est un des composés majoritaire de la matrice extracellulaire. Le collagène assure la structure fibrillaire de la matrice extracellulaire permettant le maintien mécanique du tissu cellulaire.

*Tableau 6: Bilan des résultats obtenus sur le dosage du collagène III (par dosage de type ELISA) dans les surnageant de culture de fibroblastes ayant été traités avec les différentes formules pendant 48h. Les résultats sont exprimés en quantité relative par rapport au témoin non traité*

| Quantité relative de Collagène III dosée dans le témoin non traité | Quantité relative de Collagène III dosée dans le surnageant de culture traité avec le miel seul | Quantité relative de Collagène III dosée dans le surnageant de culture traité avec le miel + HA |
|---|---|---|
| 100% | 106% | 125% |

Les effets observés avec le miel et l'acide hyaluronique sur la production de collagène III sont supérieurs aux effets induits par le miel seul. Un effet de bénéfique de cette composition est observé sur la production de collagène III par les fibroblastes.

**4. Evaluation de l'effet synergique d'un mélange miel + collagène**

*a. Prolifération fibroblastique*

Type de collagène testé = collagène bovin (dose utilisée dans la composition 5%)
Concentration en composition testée = 1,28% v/v
Durée du traitement = 48 h
Cellules testées = fibroblastes

Tableau 7

| Échantillons | Miel | Collagène | Collagène + miel |
|---|---|---|---|
| Pourcentage de prolifération fibroblastes (%) | 188% | 172% | 261% |

Un effet synergique du miel et du collagène est observé sur la prolifération des fibroblastes.

*b. Principe des tests de prolifération sur kératinocytes*

Les produits sont dilués pour obtenir une concentration de 0,1 % v/v de l'échantillon.
Le protocole opératoire est le suivant :
Une lignée cellulaire de kératinocytes est ensemencées dans des microplaques 48 puits à raison de 20 000 cellules par puits avec un volume de milieu de culture complet (10% de sérum) de 500 $\mu$L. Les cellules vont ensuite adhérer au fond des puits durant 24 heures. Le milieu sera ensuite remplacé par le produit à tester dilué à 0,1% dans du milieu de culture contenant 2,5% de sérum. Le produit est testé à raison de 500 $\mu$L par puits et incubé pendant 24 heures à 37°C + 5% $CO_2$. Les cellules sont ensuite décollées avec de la trypsine puis comptées sur lame de Malassez avec un colorant d'exclusion (bleu Trypan). Le pourcentage de prolifération kératinocytaire est déterminé par rapport à un témoin non traité avec les échantillons.

*c. Prolifération kératinocytes*

Type de collagène testé = peptide de collagène dose utilisée dans la composition 1,5%
Concentration en composition testée = 0,1% v/v
Durée du traitement = 24 h
Cellules testées = kératinocytes

*Tableau 8*

| Échantillons | Miel | Collagène | Collagène + miel |
|---|---|---|---|
| Pourcentage de prolifération kératinocytes (%) | 143% | 129% | 186% |

Un effet synergique de l'association miel et collagène est observé sur la prolifération kératinocytaire.

**5. Evaluation de l'effet synergique d'un mélange miel + béta-glucane**

*a) Prolifération kératinocytes*

Concentration en composition testée = 0,1% v/v

Durée du traitement = 24 h

Cellules testées = kératinocytes

*Tableau 9*

| Échantillons | Miel | Béta-glucanes | Miel + Béta-glucanes |
|---|---|---|---|
| Pourcentage de prolifération kératinocytes (%) | 131% | 162% | 193% |

Un effet bénéfique de l'association miel et béta-glucanes est observé sur la prolifération des kératinocytes.

*b) Résultats sur la stimulation de la synthèse de collagène III*

Le collagène III est un des composés majoritaire de la matrice cellulaire. Le collagène assure la structure fibrillaire de la matrice extracellulaire permettant le maintien mécanique du tissu cellulaire.

*Tableau 10 : bilan des résultats obtenus sur le dosage du collagène III (par dosage de type ELISA) dans les surnageant de culture de fibroblastes ayant été traités avec les différentes formules pendant 48h. Les résultats sont exprimés en quantité relative par rapport au témoin non traité.*

| Quantité relative de Collagène III dosé dans les surnageants de culture traités avec | | | |
|---|---|---|---|
| Témoin non traité | Miel seul | Béta-glucanes | Miel + Béta-glucanes |
| 100% | 106% | 88% | 119% |

La quantité de collagène III produite par les fibroblastes est notablement plus importante que celle obtenue avec le miel seul ou les béta-glucanes seuls. Ce résultat indique que le miel en mélange avec les béta-glucanes ont un effet synergique sur la production de collagène III par les fibroblastes.

**6. Evaluation de l'effet synergique d'un mélange miel + acide hyaluronique + béta-glucane**

Concentration en échantillon testée = 1,28% v/v
Durée du traitement = 48 h
Cellules testées = fibroblastes

*Tableau 11*

| Échantillons | Miel | Acide hyaluronique | Béta-glucanes | Miel + Béta-glucanes | Miel + acide hyaluronique + Béta-glucanes |
|---|---|---|---|---|---|
| Pourcentage de prolifération fibroblastes (%) | 154 | 182 | 222 | 189 | 239 |

L'association miel + acide hyaluronique + béta-glucanes induit une augmentation de la prolifération des fibroblastes par rapport aux composés seuls.

**Revendications**

1. Composition sous forme liquide ou semi-liquide comprenant :

   - entre 5% et 99,8% de miel, et
   - au moins un constituant de la matrice extracellulaire choisi parmi le collagène, l'élastine et les glycosaminoglycanes dont l'acide hyaluronique qui s'il est compris dans la composition est présent à raison de entre 0,05 et 10%, et/ou au moins un béta-glucane,

les pourcentages étant donnés en poids par rapport au poids de matière sèche de la composition, pour son utilisation comme produit de santé cicatrisant et/ou antibactérien à application topique,

2. Composition pour son utilisation selon la revendication 1 **caractérisée en ce que** le au moins un constituant de la matrice extracellulaire est l'acide hyaluronique.

3. Composition pour son utilisation selon la revendication 1, **caractérisée en ce que** le au moins un constituant de la matrice extracellulaire est un glycosaminoglycane choisi parmi les héparanes sulfates, les kératanes sulfates et les chondoïtines sulfates.

4. Composition pour son utilisation selon l'une des précédentes revendications, **caractérisée en ce que** le miel est du miel d'origine naturelle.

5. Composition pour son utilisation selon la revendication 4, **caractérisée en ce que** le miel est du miel de thym et/ou de miellat et/ou de sarrasin et/ou de manuka et/ou leurs mélanges.

6. Composition pour son utilisation selon l'une des revendications 1 à 4, **caractérisée en ce que** le miel est du miel artificiel.

7. Composition pour son utilisation selon la revendication 6, **caractérisée en ce que** le miel artificiel est constitué majoritairement de glucose et/ou fructose et/ou saccharose.

8. Composition pour son utilisation selon l'une des précédentes revendications, **caractérisée en ce qu'**elle se présente sous forme de gel, pommade, spray, crème, émulsion ou ovule.

9. Composition pour son utilisation selon l'une des précédentes revendications, **caractérisée en ce qu'**elle a été traitée par irradiation gamma.

10. Composition pour son utilisation selon l'une des précédentes revendications pour son utilisation comme dispositif médical destiné au traitement des plaies.

11. Composition pour son utilisation selon la précédente revendication pour son application comme dispositif médical destiné au traitement des brûlures de premier et second degré, au traitement et/ou à la prévention des désunions post opératoires de cicatrice, au traitement et/ou à la prévention post-opératoire des cavités résiduelles des sinus pilonidaux, au traitement des cicatrices chirurgicales infectées après mise à plat, au traitement des ulcères et escarres, au traitement des plaies traumatiques, au traitement des plaies aigües et chroniques, au traitement des plaies cancérologiques, au traitement des emplacements de stomies, au traitement des dermabrasions, et/ou au traitement des plaies superficielles.

12. Composition pour son utilisation selon l'une des précédentes revendications pour son utilisation comme composition pour le traitement des dermatites, de l'acné, des érythèmes fessiers, de l'impétigo, de furoncles, de panaris ou de mycoses, comme gel buccal destiné à traiter les aphtes et les mucites, stick ou baume à lèvre destiné à traiter les gerçures, crème pour les mains et les pieds destinée notamment à la réparation ou au traitement des crevasses et des engelures, baume destiné à la prévention et/ou au traitement des crevasses du mamelon, gel vaginal ou suppositoire ou crème rectale.

13. Composition pour son utilisation selon l'une des précédentes revendications, **caractérisée en ce qu'**elle comprend entre 0,05 et 1% d'acide hyaluronique en poids de matière sèche.

14. Composition pour son utilisation selon l'une des précédentes revendications, **caractérisée en ce qu'**elle comprend de l'acide hyaluronique de poids moléculaire supérieur à 100 000 Da.

15. Composition pour son utilisation selon l'une des précédentes revendications, **caractérisée en ce qu'**elle comprend de l'acide hyaluronique et au moins un béta-glucane.

**16.** Composition pour son utilisation selon l'une des précédentes revendications, **caractérisée en ce qu'**elle comprend des béta-glucanes constitués d'unités glucose reliées par des liaisons β(1,3) et/ou β(1,4) et/ou β(1,6).

**17.** Composition pour son utilisation selon l'une des précédentes revendications, **caractérisée en ce qu'**elle comprend entre 0,01% et 5% de béta-glucanes en poids de matière sèche.

**18.** Composition pour son utilisation selon l'une des précédentes revendications, **caractérisée en ce qu'**elle comprend entre 0,001% et 50% de collagène en poids de matière sèche.

**Patentansprüche**

**1.** Zusammensetzung in flüssiger oder halbflüssiger Form mit:

- zwischen 5 % und 99,8 % Honig, und
- wenigstens einem Bestandteil der extrazellulären Matrix, der aus Kollagen, Elastin, und den Glykosaminglykanen darunter Hyaluronsäure ausgewählt ist, die, wenn diese in der Zusammensetzung vorhanden ist, mit 0,05 bis 10 % vorliegt, und/oder wenigstens einem Beta-Glucan,

wobei die Prozentangaben sich auf das Gewicht bezüglich der Trockenmasse der Zusammensetzung beziehen, zu deren Verwendung als vernarbendes und/oder antibakterielles Gesundheitsprodukt für die topischen Anwendung.

**2.** Zusammensetzung zu deren Verwendung nach Anspruch 1, **dadurch gekennzeichnet, dass** der wenigstens eine Bestandteil der extrazellulären Matrix Hyaluronsäure ist.

**3.** Zusammensetzung zu deren Verwendung nach Anspruch 1, **dadurch gekennzeichnet, dass** der wenigstens eine Bestandteil der extrazellulären Matrix ein Glykosaminoglykan ist, das unter den Heparansulfaten, den Keratansulfaten und den Chondroitinsulfaten ausgewählt ist.

**4.** Zusammensetzung zu deren Verwendung nach einem der vorhergehenden Ansprüche, **dadurch gekennzeichnet, dass** der Honig Honig natürlicher Herkunft ist.

**5.** Zusammensetzung zu deren Verwendung nach Anspruch 4, **dadurch gekennzeichnet, dass** der Honig Honig von Thymian und/oder Honigtau und/oder Buchweizen und/oder Manuka und/oder deren Mischung ist.

**6.** Zusammensetzung zu deren Verwendung nach einem der Ansprüche 1 bis 4, **dadurch gekennzeichnet, dass** der Honig künstlicher Honig ist.

**7.** Zusammensetzung zu deren Verwendung nach Anspruch 6, **dadurch gekennzeichnet, dass** der künstliche Honig überwiegend aus Glukose und/oder Fruktose und/oder Saccharose gebildet ist.

**8.** Zusammensetzung zu deren Verwendung nach einem der vorhergehenden Ansprüche, **dadurch gekennzeichnet, dass** diese in der Gestalt eines Gels, einer Salbe, eines Sprays, einer Creme, einer Emulsion oder als Zäpfchen vorliegt.

**9.** Zusammensetzung zu deren Verwendung nach einem der vorhergehenden Ansprüche, **dadurch gekennzeichnet, dass** diese mit Gammastrahlung behandelt worden ist.

**10.** Zusammensetzung zu deren Verwendung nach einem der vorhergehenden Ansprüche zu deren Verwendung als medizinisches Mittel zur Behandlung von Wunden.

**11.** Zusammensetzung zu deren Verwendung nach dem vorhergehenden Anspruch zu deren Anwendung als medizinisches Mittel zur Behandlung von Verbrennungen ersten und zweiten Grades, zur Behandlung und/oder Prävention der postoperativen Öffnungen von Narben, zur Behandlung und/oder postoperativen Prävention von Restkavitäten des Pilonidalsinus, zur Behandlung von chirurgischen Narben, die sich nach dem Flachlegen infiziert haben, zur Behandlung von Geschwüren und Dekubiti, zur Behandlung von traumatischen Wunden, zur Behandlung von akuten oder chronischen Wunden, zur Behandlung von Krebswunden, zur Behandlung der Stelle von Stomata, zur Behandlung von Dermabrasionen und/oder zur Behandlung von oberflächlichen Wunden.

**12.** Zusammensetzung zu deren Verwendung nach einem der vorhergehenden Ansprüche zu deren Verwendung als Zusammensetzung für die Behandlung von Hautentzündungen, Akne, von Windelausschlag, Impetigo, Furunkeln, Panaritium, Mykosen, als Mundgel zur Behandlung von Mundfäule und Mukositis, als Stift oder Balsam für die Lippe zur Behandlung von Rissen, als Creme für die Füße oder Hände, insbesondere zur Behebung oder Behandlung von Spalten und Erfrierungen, als Balsam für die Prävention und/oder Behandlung von Brustwarzenrissen, als vaginales Gel oder Zäpfchen oder Rektalcreme.

**13.** Zusammensetzung zu deren Verwendung nach einem der vorhergehenden Ansprüche, **dadurch gekennzeichnet, dass** diese zwischen 0,05 und 1 % Trockenmasse Hyaluronsäure aufweist.

**14.** Zusammensetzung zu deren Verwendung nach einem der vorhergehenden Ansprüche, **dadurch gekennzeichnet, dass** diese Hyaluronsäure mit einem Molekulargewicht von 100.000 Da aufweist.

**15.** Zusammensetzung zu deren Verwendung nach einem der vorhergehenden Ansprüche, **dadurch gekennzeichnet, dass** diese Hyaluronsäure und wenigstens ein Beta-Glucan aufweist.

**16.** Zusammensetzung zu deren Verwendung nach einem der vorhergehenden Ansprüche, **dadurch gekennzeichnet, dass** diese Beta-Glucane aufweist, die von Glukoseeinheiten gebildet sind, die durch die Bindung β(1,3) und/oder β(1,4) und/oder β(1,6) verbunden sind.

**17.** Zusammensetzung zu deren Verwendung nach einem der vorhergehenden Ansprüche, **dadurch gekennzeichnet, dass** diese zwischen 0,01 % und 5 % Trockenmasse an Beta-Glucan aufweist.

**18.** Zusammensetzung zu deren Verwendung nach einem der vorhergehenden Ansprüche, **dadurch gekennzeichnet, dass** diese zwischen 0,001 % und 50 % Trockenmasse Kollagen aufweist.

**Claims**

**1.** A composition in liquid or semi-liquid form, comprising:

- between 5% and 99.8% honey, and
- at least one constituent of the extracellular matrix chosen from collagen, elastin and glycosaminoglycans, including hyaluronic acid, which, if it is included in the composition, is present at between 0.05% and 10%, and/or at least one beta-glucan,

the percentages being given by weight with respect to the weight of dry matter of the composition,
for use thereof as a healing and/or antibacterial health product for topical application.

**2.** A composition for use thereof according to claim 1, **characterised in that** the at least one constituent of the extracellular matrix is hyaluronic acid.

**3.** A composition for use thereof according to claim 1, **characterised in that** the at least one constituent of the extracellular matrix is a glycosaminoglycan chosen from heparan sulphates, keratan sulphates and chondroitin sulphates.

**4.** A composition for use thereof according to any of the preceding claims, **characterised in that** the honey is honey of natural origin.

**5.** A composition for use thereof according to claim 4, **characterised in that** the honey is thyme and/or honeydew and/or buckwheat and/or manuka honey and/or mixtures thereof.

**6.** A composition for use thereof according to any of claims 1 to 4, **characterised in that** the honey is artificial honey.

**7.** A composition for use thereof according to claim 6, **characterised in that** the artificial honey consists mainly of glucose and/or fructose and/or saccharose.

**8.** A composition for use thereof according to any of the preceding claims, **characterised in that** it is in gel, ointment, spray, cream, emulsion or pessary form.

9. A composition for use thereof according to any of the preceding claims, **characterised in that** it has been treated by gamma irradiation.

10. A composition for use thereof according to any of the preceding claims, for use thereof as a medical device intended for the treatment of wounds.

11. A composition for use thereof according to the preceding claim, for application thereof as a medical device intended for the treatment of first- and second-degree burns, for the treatment and/or prevention of post-operative scar separations, for the post-operative treatment and/or prevention of residual cavities of the pilonidal sinuses, for the treatment of infected surgical scars after flattening, for the treatment of ulcers and scabs, for the treatment of traumatic wounds, for the treatment of acute and chronic wounds, for the treatment of cancerological wounds, for the treatment of stoma sites, for the treatment of skin abrasions, and/or for the treatment of superficial wounds.

12. A composition for use thereof according to any of the preceding claims, for use thereof as a composition for the treatment of dermatitis, acne, gluteal erythema, impetigo, furoncles, whitlows or mycoses, as a buccal gel intended for treating mouth ulcers and mucositis, a lipstick or lip salve intended for treating chapping, cream for the hands and feet intended in particular for the repair or treatment of cracks and chilblains, a balm intended for the prevention and/or treatment of nipple cracks, vaginal gel or suppository or rectal cream.

13. A composition for use thereof according to any of the preceding claims, **characterised in that** it comprises between 0.05% and 1% hyaluronic acid by weight of dry matter.

14. A composition for use thereof according to any of the preceding claims, **characterised in that** it comprises hyaluronic acid with a molecular weight greater than 100,000 Da.

15. A composition for use thereof according to any of the preceding claims, **characterised in that** it comprises hyaluronic acid and at least one beta-glucan.

16. A composition for use thereof according to any of the preceding claims, **characterised in that** it comprises beta-glucans comprising glucose units connected by $\beta(1,3)$ and/or $\beta(1,4)$ and/or $\beta(1,6)$ bonds.

17. A composition for use thereof according to any of the preceding claims, **characterised in that** it comprises between 0.01% and 5% beta-glucans by weight of dry matter.

18. A composition for use thereof according to any of the preceding claims, **characterised in that** it comprises between 0.001% and 50% collagen by weight of dry matter.

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- CH 699182
- US 6630442 B
- US 2003021834 A
- US 201110427 B
- FR 1258722

**Littérature non-brevet citée dans la description**

- **COOPER R A et al.** Antibacterial activity of honey against strains of Staphylococcus aureus from infected wounds. Journal of the Royal Society of Medecine, 01 Juin 1999, vol. 92
- **ALANDEJANI T et al.** Effectiveness of honey on Staplylococcus aureus and Pseudomonas aeruginosa biofilms. *Otolaryngology and head and neck surgery,* 01 Juillet 2009, vol. 141
- Potential of honey in the treatment of wounds and burns. **MOLAN P C.** ADIS. American Journal of Clinical Dermatology, 01 Février 2001, vol. 2
- **SOLIDATI D et al.** Mucosal wound healing after nasal surgery. A controlled clinical trial on the efficacy of hyaluronic acid containing cream. *Drugs under experimental and clinical research,* vol. 25 (6), 253-261
- **LEON-VILLAPALOS J et al.** Topical management of facial burns. *Burns,* 01 Novembre 2008, vol. 34 (7), 903-911
- Cosmeceuticals and natural products : wound healing. **DAVIS S C et al.** Clinics in Dermatology. J.B. Lippincott, 01 Septembre 2009, vol. 27
- **SINGH ONKARET.** Collagen dressing versus conventional dressings in burn and chronic wounds : a rétrospective study. *Journal of Cutaneousand Aesthetic Surgery,* Janvier 2011, vol. 4 (1